# EUROPEAN PATENT APPLICATION

(11) **EP 2 491 905 A1**
(43) Date of publication of application: **29.08.2012**
(21) Application number: 12157257.2
(22) Date of filing: 28.02.2012
(51) Int. Cl.: A61F 13/02

(54) **Adhesive Bandage**

(30) Priority: 28.02.2011 US 201113036471
(71) Applicant: Johnson & Johnson Consumer Companies Inc., Skillman, NJ 08558 (US)
(72) Inventor: Chan, Jennifer Wing-Yee, Belle Mead, NJ New Jersey 08502 (US); Eknoian, Michael W., Warren, NJ New Jersey 07059 (US); Reddy, Megha, Princeton, NJ New Jersey 08540 (US); Rizzo, Carmine Michael, Cliffwood Beach, NJ New Jersey 07735 (US); Petersack, Vincent John, Eastampton, NJ New Jersey 08060 (US)
(74) Representative: Carpmaels & Ransford

(57) **Abstract**

Adhesive bandages having a backing layer with first and second opposing surfaces, and an embossed absorbent pad associated with the backing layer, the embossed absorbent pad including a first surface that faces the second surface of the backing layer and an opposing second surface having a plurality of tufted regions surrounded and separated by a first network of interconnecting channels recessed in the second surface of the absorbent pad; where the tufted regions have a substantially uniform first density, the channel base region has a substantially uniform second density that is greater than the substantially uniform first density of the tufted regions and the network of channels is visible.

## Description

### FIELD OF THE INVENTION

The present invention relates to an adhesive bandage to be applied onto the skin, particularly an adhesive bandage with an absorbent pad having tufted regions surrounded and separated by a network of interconnecting channels recessed in the surface of the absorbent pad.

### BACKGROUND OF THE INVENTION

There are many types of wounds to the human body. They may be open or closed. Open wounds include incisions or incised wounds, lacerations, abrasions, puncture wounds and penetration wounds. Closed wounds include contusions (bruises), hematomas and crushing injuries. Depending on the severity of the wound, certain wounds may require closing via sutures and the like, followed by topical application of a wound dressing to protect the wound from dirt and further damage by contact. Similarly, wounds due to medical surgical procedures typically require application of a wound dressing subsequent to surgery to protect the wound.

As there are different types of wounds to the body, so are there different types of dressings for application to such wounds. In certain dressings, absorbent structures, e.g. pads, may be used to absorb exudates from the wound. Other wound dressings may be free of such absorbent pads. For example, US 3,053,252 discloses bandages where non-absorbent support surfaces or edges project beyond the absorbent surface towards the wound site to form an embossed pattern of absorbent areas and non-absorbent support surfaces. US 4,781,710 discloses bandages that utilize pads having tufted regions surrounded by channels. The channels require both a transport region and a storage region, where density of the transport region is greater than that of the storage region. US 4,259,387 relates to absorbent products adapted to absorb body fluids. Such products utilize a loosely compacted cellulosic fibrous batt having a plurality of spaced, relatively narrow, dense lines in the general plane of the batt. The batts have a nonuniform density where the areas of the batt adjacent the lines cover or obscure the lines due to modified batt regions of lesser density adjacent the lines than the average density of the batt.

### SUMMARY OF THE INVENTION

The present invention relates to bandages for application to abrasions or cuts in the skin, where the bandage includes a backing layer having a first surface and a second surface opposite the first surface; and an absorbent pad associated with the backing layer. The absorbent pad includes a first surface facing the backing layer and that has a first surface area, and a second surface opposite the first surface and that has a second surface area. The absorbent pad comprises a plurality of tufted regions surrounded and separated by a first network of interconnecting channels recessed in the second surface of the absorbent pad. The channels comprise and are defined by channel sidewalls extending away from the second surface and into the core body of the absorbent pad and terminating in a first surface of a channel base region extending between the channel sidewalls. The absorbent pad has a thickness defined by the distance between the first and second surfaces of the absorbent pad. The tufted regions have a substantially uniform first density, while the channel base region has a substantially uniform second density that is greater than the substantially uniform first density of the tufted regions. The channel base region has a thickness defined by the distance between the first surface of the channel base region and a second surface of the channel base region opposite the first surface. The network of interconnecting channels is visible to the user of the bandages.

### BRIEF DESCRIPTION OF THE DRAWINGS

The present invention will now be described in greater detail with reference to embodiments represented in the drawings.
FIG. 1 is a perspective view of an embodiment of an adhesive bandage of the present invention;
FIG. 2a is a cross-sectional view of the adhesive bandage illustrated in FIG. 1 taken along line 2a---2a;
FIG. 2b is a cross-sectional side view of an adhesive bandage similar to the bandage as shown in FIG. 1, where the second surface of the absorbent pad comprises a covering layer;
FIG. 3 a is a view of a first embodiment of the absorbent pad depicted in FIG. 1 taken perpendicular to the second surface of the bandage;
FIG. 3b is a cross-sectional side view taken along line 3b---3b of the pad embodiment illustrated in FIG. 3 a;
FIG. 4a is a cross-sectional side view of a second embodiment of an absorbent pad used in bandages of the present invention;
FIG. 4b is a cross-sectional side view of one embodiment of a bandage according to the present invention utilizing the absorbent pad depicted in FIG. 4a;
FIG. 4c is a cross-sectional side view of another embodiment of a bandage according to the present invention utilizing the absorbent pad depicted in FIG. 4a;
FIG. 4d is a perspective view of the bandage depicted in FIG. 4c as seen from the first side of the backing layer of the bandage;
FIG. 5 is a view of another embodiment of an absorbent pad used in bandages of the present invention;
FIG. 6 is a cross-sectional side view of another embodiment of an adhesive bandage of the present invention; and.
FIG. 7 is a view of another embodiment of an absorbent pad used in bandages of the present invention.

### DETAILED DESCRIPTION OF THE INVENTION

As used herein, the word "comprising" is intended to cover also "comprising essentially of' and "consisting of".

Bandages according to the present invention include a backing layer having a first surface facing away from the skin and a second surface, opposite the first surface, and facing the skin. The backing layer may have various shapes, e.g. rectangular, square, oval, circular, ovoid, oblong, etc. The shape of the bandage is defined by the shape of the backing layer. The backing layer may be thin, highly flexible or deformable, water-impervious, and clear or opaque. In general, the backing layer's thickness should fall within the range of 0.05 to 0.20 millimeter to achieve the forming and flexing characteristics desired.

A polyethylene film may be used as the backing layer, and particularly effective results may be achieved with stretchable, elastomeric films formed of polyurethane, which has the further advantage of gas (including water vapor) transmissibility. It is to be understood, however, that other flexible, water insoluble polymeric films known in the art may be used. Furthermore, the backing layer may be formed from closed-cell polymeric foam, particularly one with an integral skin covering the side facing away from the skin of the user. Foam layers formed of polyurethane or polyethylene are suitable, while other polymeric foams having similar properties may be used. In addition, the backing layer may be made from other polyolefins, vinyl polyethylene acetate, textile non-woven fabrics, rubber, or other materials known in the bandage art. Polymers used to make backing layers used in bandages of the present invention may exhibit viscosity of about 500 to 500,000 centipoises at temperatures of about 190°C, or about 1,000 to 30,000 centipoises at temperatures of about 190°C, or about 3,000 to 15,000 centipoises at temperatures of about 190°C. The backing layer may be impermeable to liquid, but permeable to gas, which allows the wound and the skin to which the bandage of the present invention is adhered to breathe. The backing layer may have pores of such a size that will allow only the passage of gases, which have molecules of extremely small size. Finally, one can conceive of a backing layer that is perforated for more ventilation of the skin. Perforations may be circular in area and have a range of diameters, such as from about 0.1 to about 0.8 millimeters. However, the backing layer may be totally impermeable to gases, when necessary.

Bandages of the present invention comprise an absorbent pad associated with the backing layer. As used herein, "associated with the backing layer" means that the absorbent pad is affixed either directly or indirectly to the backing layer so that it will not become separated from the backing layer during normal use. Association may be accomplished by applying an adhesive layer between the second surface of the backing layer and first surface of the absorbent pad, thereby adhesively bonding the absorbent pad directly to the backing layer. The absorbent pad also may be associated with an intermediate layer, which in turn is associated with the backing layer, thus indirectly associating the absorbent pad to the backing layer via the intermediate layer. Association also may be accomplished by other known means such as ultrasonic welding.

The absorbent pad includes a first surface facing the second surface of the backing layer, and that has a first surface area, and a second surface opposite the first surface and facing the skin, and that has a second surface area. The absorbent pad used in bandages of the present invention may have various shapes, e.g. rectangular, square, oval, circular, ovoid, oblong, etc., and is sized to cover less area than the backing layer so that, in use, the adhesive layer on the backing layer is in contact with the user's skin, but preferably does not contact the wound surface. The absorbent pad may be a fibrous matrix comprising materials selected from the group consisting of threads, yarns, nets, laces, felts and nonwovens. The basis weight of the materials selected as the absorbent pad in bandages of the present invention may be any of those used conventionally to make absorbent pads for bandages utilized in applications similar to those of the present invention. For example, the basis weight of the material may be from about 3.0 oz/yd² to about 5.5 oz/yd², although the invention is not limited as such.

The absorbent pad may be made from any type of material commonly used in the art in forming such pads for use in bandages. Materials that may be used include cellulose fiber gauzes, lightly cross-linked tissue structures, and other fibrous pads comprising natural or synthetic materials such as cotton, polyethylene terepthalate (PET), polypropylene (PP), or blends of viscose rayon and polyolefins, or other materials that are absorbent and that are capable of being embossed to form the network of interconnecting channels recessed in the surface of the absorbent pad. The absorbable pad may be not degradable in water.

The absorbent pad comprises a plurality of tufted regions surrounded and separated by a first network of interconnecting channels recessed in the second surface of the absorbent pad. Tufted regions are understood to be substantially undensified, i.e. relatively low density regions compared to the density of the channel base regions. The term "channels" refers to those recessed regions which separate and surround the tufted regions. The first and second surfaces of the tufted regions are coincident with the first and second surfaces of the absorbent pad, respectively. The absorbent pad has a thickness defined by the maximum distance between the first and second surfaces of the absorbent pad. Typically, the thickness of the absorbent pad may range from about 0.3 millimeters to about 2.5 millimeters.

The interconnecting channels recessed in the second surface of the absorbent pad comprise and are defined by channel sidewalls extending away from the second surface and into the core body of the absorbent pad and terminating in a first surface of a channel base region extending between the channel sidewalls. The channel base region further has a second surface opposite the first surface, and a thickness defined by the distance between the first and second surfaces of the channel base region. A second network of interconnecting channels may be recessed into the first surface of the absorbent pad. The second network of interconnected channels may be in alignment with the first network of interconnecting channels found in the second surface of the absorbent pad. The channels in the first surface of the absorbent pad comprise and are defined by channel sidewalls extending away from the first surface and into the core of the absorbent pad and terminating in the second surface of the channel base region, which extends between the channel sidewalls in the first surface of the absorbent pad.

The tufted regions have a substantially uniform first density. By "substantially uniform first density", it is meant that the density of the tufted region adjacent the channels is substantially the same as the average density of the tufted region across the entire area of the tufted region, such that the channels are not obscured by the tufted region adjacent the channel. The channel base region has a substantially uniform second density that is greater than the substantially uniform first density of the tufted regions. By "substantially uniform second density", it is meant that the density of the channel base regions is substantially the same throughout the channel base region. The second density of the channel base region may be about 12 times or less greater than the first density of the tufted region, or from 1.1 to about 10 times greater than the density of the tufted regions. The substantially uniform first density of the tufted regions may be from about 0.082 grams/cubic centimeter to about 0.17 grams/cubic centimeter. The substantially uniform second density of the channel base regions may be from about 0.11 grams/cubic centimeter to about 0.96 grams/cubic centimeter. The network of recessed, interconnecting channels may comprise from about 5 to about 20 percent of the surface area of the surface of the absorbent pad, or from about 10 to about 15 percent of the surface area of the surface of the absorbent pad. This is whether the channels are present on one surface or both surfaces of the pad.

The thickness of the channel base region may vary from about 0.1 millimeters to about 1.5 millimeters, depending on the depth of the channels and whether both surfaces or only one surface of the absorbent pad comprise channels recessed therein. In addition to the substantially uniform first density of the tufted regions, the channels have a width and depth such that the network of interconnecting channels is visible to the user. By "visible", it is meant that the network of interconnecting channels is distinguishable from the tufted regions when the bandage is observed by the user at distances normally encountered when applying the bandage to the skin or wound, e.g. from about 6 to about 24 inches, or from about 6 to about 12 inches.

Interconnecting channels in the absorbent pad serve a number of functions. Interconnecting channels function to transport low viscosity fluid, e.g. wound exudates, away from the wound surface by wicking action. As used herein, low viscosity fluids are those with viscosities of less than 50,000 centipoises. The channel base regions, due to their high density relative to the tufted regions, have smaller capillaries between their fibers and are not able to absorb as much fluid as the lower density tufted regions. Thus, channel base regions provide relatively fast wicking.

The tufted regions have a density less than the density of the channel base regions and, therefore, have larger capillaries between their fibers than the channel base regions. This gives the tufted regions the tendency to absorb more liquid than the channel base regions. In use, low viscosity liquid which is deposited onto the absorbent pad is absorbed to some degree by the tufted regions. In bandages of the present invention, tufted regions adjacent to the area where the low viscosity liquid is deposited may not be able to absorb all liquid fast enough to prevent "puddling" of the liquid on the absorbent pad, which resultantly may give the user an uncomfortably wet feeling or cause skin irritation. The excess liquid may enter the interconnecting channels, which may then direct the liquid to other tufted regions of the absorbent pad that may be less saturated, such that they will accept and absorb the excess low viscosity liquid.

Another function of the interconnecting channels is to serve as a storage depot for high viscosity fluids. As used herein, high viscosity fluids are those with viscosities of greater than 50,000 centipoises. If a user places a high viscosity ointment, balm, emollient, unguent, cream or salve on the wound site, and then places a standard pad with a uniform density throughout its structure in contact with the high viscosity fluid, the fluid may spread from the wound site to portions of the pad that do not cover the wound. If, however, a user places a high viscosity ointment, balm, emollient, unguent, cream or salve on the wound site, and then places bandages of the present invention in contact with the high viscosity fluid, the fluid may flow from the lower density tufted regions to the relatively higher density channel base regions in the interconnecting channels. In this way, the high viscosity fluid may be maintained at the site of the wound.

Yet another function of the network of interconnecting channels is to provide a visual cue to the user. The term "visual cue", as used herein, refers to visual information, e.g. the way a product or portion of a product appears to the user, which is used in connection with identifying a function or functions of the product. With respect to bandages of the present invention, the visual cue is as to the function of the channels in transporting low viscosity fluid away from the wound surface by wicking action, and/or in serving as a storage depot for high viscosity fluids. Accordingly, the network of interconnecting channels is visible, as defined above. The width of the channels may be from about 0.2 millimeters to about 0.6 millimeters or from about 0.3 millimeters to about 0.45 millimeters, and the depth is from about 0.02 to about 2.0 millimeters, or from about 0.04 to about 1.1 millimeters. At least the first network of interconnecting channels recessed in the second surface of the absorbent pad may be visible to the user when observed from the second surface of the absorbent pad. Both first and second networks of interconnecting channels recessed in the first and second surfaces of the absorbent pad, respectively, may be visible to the user when observed from the respective surface. The second network of interconnecting channels recessed in the first surface of the absorbent pad may be visible to the user through the backing layer, where the backing layer is transparent or translucent. The backing layer may conform to the network of interconnecting channels due to the process for making the bandage. In this embodiment, the backing layer itself comprises the network of interconnecting channels recessed in the first surface of the backing layer, as shown herein.

The present invention the bandage may further comprise an intermediate layer disposed between the backing layer and the absorbent pad. The intermediate layer typically may be used where the backing layer is transparent or translucent. In such bandages, the intermediate layer may be of a color similar to skin of the user and may be used to mask the appearance of the absorbent pad to the user, which pad may include exudates from the wound, including blood. When used, such intermediate layers may be made from materials known to those skilled in the art for similar use. The intermediate layer can be made from any type of material commonly used in the art in forming adhesive bandages. The intermediate layer can be made from, for example, plastic netting materials such as those sold under the tradename Delnet, available from Delstar Technologies, Inc., Middletown, DE.

The second surface, i.e. the skin-facing surface, of the absorbent pad may comprise a covering layer affixed thereto. When present, the covering layer is considered as an integral component of the second surface of the absorbent pad and the network of interconnected channels is embossed into the absorbent pad after application of the covering layer to the absorbent pad. As such, the covering layer also comprises the network of interconnecting channels recessed in the skin-facing surface. The covering layer provides additional protection to the wound and, where the absorbent pad is fibrous, prevents fibers from sticking to the wound or obscuring the channels. The covering layer is permeable to exudates to allow passage of the exudates from the wound to the absorbent pad. Thus, the covering layer may include perforations. The covering layer may be made of, for example, plastic netting materials such as those sold under the tradename Delnet, available from Delstar Technologies, Inc., Middletown, DE.

In general, any of a variety of pressure-sensitive adhesives can be utilized in the present invention as the adhesive layer to bond the absorbent pad to the backing layer and to adhere the bandage to the skin. In particular, pressure-sensitive adhesives that are biocompatible with human skin are typically utilized. Moreover, an adhesive used in the present invention may be either generally water soluble, or generally insoluble or dispersible in an aqueous environment. For instance, one commercially available dispersible pressure-sensitive adhesive is sold under the trade name of HL-9415-X and is available from H.B. Fuller Company. Another suitable adhesive includes about 10-75% by weight of a polyalkyloxazoline polymer, 10-75% by weight of a functional diluent comprising a hydroxy compound or a carboxylic acid compound, and 5-50% by weight of a tackifier.

The water-dispersible polymeric component can include, for example, surfactants such as poly(ethylene oxide) alkylphenyl ethers, such as those sold under the trade names IGEPAL.CO and IGEPAL.CA (available from Rhone-Poulenc, Inc.); poly(ethylene oxide) lauryl, cetyl, and oleyl ethers such as those sold under the trade name BRIJ (available from ICI Americas, Inc.); poly(ethylene oxide) laurate; poly(ethylene oxide) oleate; sorbitan oleate; ethylene oxide/propylene oxide block copolymers such as those sold under the trade names PLURONIC and TETRONIC (available from BASF Corporation); and organic phosphate esters, such as those sold under the trade name GAFAC PE-510 (available from International Specialty Products). Examples of other components include, but are not limited to, poly(acrylic acid); poly(vinyl alcohol); poly(N-vinyl pyrrolidone); poly(acrylamide); poly(alkoxyalkyl (meth)acrylates), such as 2-ethoxy ethyl acrylate, 2-ethoxy ethyl methacrylate, 2-(2-ethoxyethoxy) ethyl acrylate, and 2-methoxy ethyl acrylate (available from SARTOMER Company, Inc.); poly(vinyl methyl ether); poly(vinyl methyl ether: maleic anhydride), sold under the trade name GANTREZ (available from International Specialty Products); poly(ether polyols), such as poly(propylene glycol) and the like, such as those sold under the trade name SANNIX (available from Sanyo Chemical Industries); copolymers thereof, and the like. Copolymers of these and alkyl (meth)acrylate esters or vinyl esters are also suitable. Gums such as those derived from okra and guar may also be used.

Still another suitable pressure-sensitive adhesive includes about 10% to about 80%, by weight, of an alkali soluble polymer; about 0 to about 30%, by weight, of a poly(vinyl methyl ether); about 30% to about 70%, by weight, of a tackifying resin; and about 5% to about 30%, by weight, of a suitable plasticizer. Still other examples of suitable adhesives include HX 9236-01 or HX 9237-01 hot melt adhesives, which are obtainable from ATO Findley, Inc.

The adhesive layer used in the present invention may comprise hydrocolloids. The hydrocolloid element used may be any substance that exhibits good performance in this utilization, as for example, sodium carboxymethylcellulose, pectin, xanthan gum, polysaccharides, sodium or calcium alginates, chitosan, seaweed extract (cageenan), polyaspartic acid, polyglutamic acid, hyaluronic acid or salts and derivatives thereof, among others.

Hydrocolloids, just as sodium carboxymethylcellulose and pectin, among others, are agents that form gels as soon as they come into contact with the bodily fluids from the wound. When used in adhesive bandages, these hydrocolloids are combined with elastomers and/or adhesives. Preferably, the adhesive bandage should provide a humid environment suitable for acceleration of the healing, but without saturation or cicatrisation.

Pectin is a complex-structure polysaccharide extracted from vegetable species, for example, peels from citric fruits or apple pulp, which has a highly hydrophilic structure. As a result, pectin associates easily with the water molecules of the bodily fluids from the wound, forming a viscous gel on the injury bed. Its chemical similarity with alginates causes the physical properties of absorption and gel formation to resemble each other.

Carboxymethylcellulose, in turn, is a cellulose derivative formed by reaction of cellulose with alkalis, such as, for example, sodium, potassium, calcium, etc., hydroxide. It is the nature of combined alkali that imparts the ionic characteristic of carboxymethylcellulose. When sodium hydroxide is used, sodium carboxymethylcellulose is formed. Just as in the case of pectin, carboxymethylcellulose dissolves rapidly in the water coming from the liquids that emanate from the wound, forming a gel on the wound with controlled viscosity.

As an additional advantage of the use of hydrocolloids, it should be noted that both pectin and carboxymethylcellulose form a gel with acidic characteristics (pH of about 4), functioning as a bactericidal agent.

The adhesive element used may be any conventional adhesive know for such use, as for example pressure acrylic adhesives, among others. Additionally, such an adhesive may contain a resin for increasing adhesion, a cohesion increasing agent, an absorption agent, preferably a polyacrylate superabsorbent, a polyacrylate salt superabsorbent or a mixture thereof, a plasticizer and optionally a pigment. The adhesive layer may further be configured in discontinuous patterns, arranged on the surface of the backing layer in lines, screen, spray or any other pattern which a person skilled in the art understands to be discontinuous.

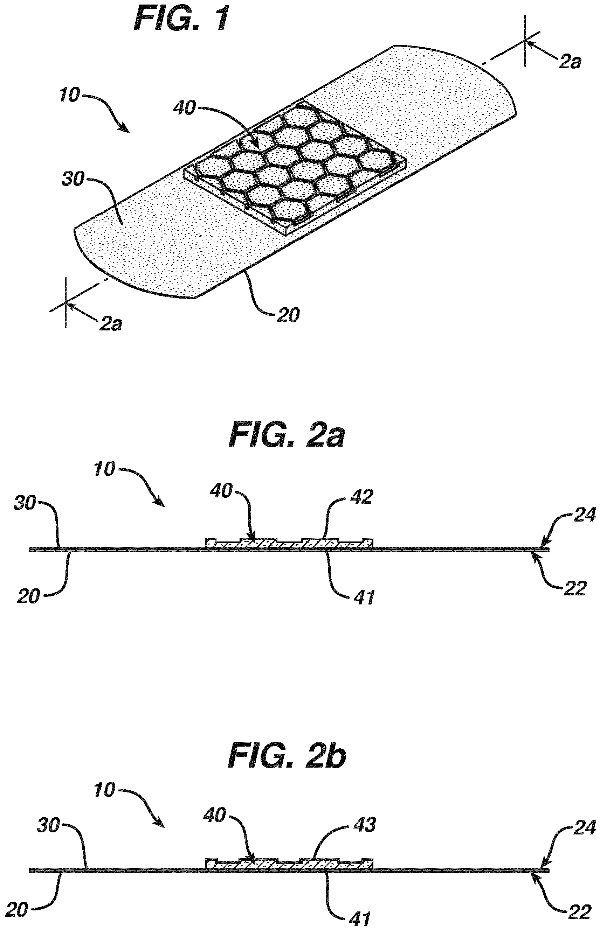

FIGS. 1 and 2a illustrate a first embodiment of an adhesive bandage of the present invention. Adhesive bandage **10** comprises backing layer **20** having first surface **22** and opposing second surface **24** and a core body bounded by first **22** and second **24** surfaces. Adhesive layer **30** is disposed on at least a portion of second surface **24** of backing layer **20.** FIGS. 1 and 2a show adhesive layer **30** disposed on the entirety of second surface **24** of backing layer **20.** Absorbent pad **40** is disposed on adhesive layer **30** such that absorbent pad **40** is associated with, e.g. by adhesive bonding, backing layer **20.** While adhesive layer **30** is shown covering the entirety of second surface **24** of backing layer **20,** it is to be understood that the adhesive layer may be disposed on a portion of the second surface of the backing layer, provided that the amount of adhesive applied, the location of the adhesive layer on the backing layer and the surface area of the portion of the second surface covered by the adhesive layer is sufficient to associate the absorbent pad to the backing layer, as discussed herein above, and to adhere the bandage to the skin.

FIG. 2b is an embodiment similar to that depicted in FIGS. 1 and 2a, except that the first surface of absorbent pad **40** comprises covering layer **43** affixed thereto. Covering layer **43** is applied to absorbent pad **40** prior to embossing, such that the network of interconnecting channels is recessed and visible in covering layer **43.**

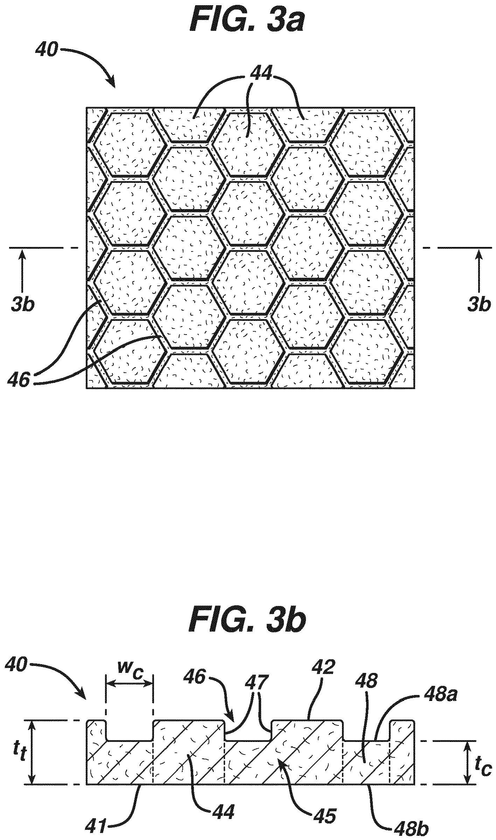

FIG. 3 a illustrates a view of absorbent pad **40** as shown in FIG. 1 as viewed perpendicular to second surface **24** of absorbent pad **40.** Pad **40** comprises tufted regions **44** surrounded and separated by a network of interconnecting channels **46** recessed in second surface **42** of pad **40,** thus providing a first embodiment of a densification pattern, e.g. a hexagon, used in bandages of the present invention.

FIG. 3b is a cross-sectional side view of absorbent pad **40** taken along line 3b-3b of FIG. 3. Absorbent pad **40** has first surface **41,** second surface **42,** core body **45,** a plurality of tufted regions **44,** and a network of interconnecting channels **46** recessed in second surface **42** of pad **40.** The thickness (tₜ) of absorbent pad **40** is defined by the distance between first **41** and second **42** surfaces of absorbent pad **40.**

Tufted regions **44** have a thickness of tₜ and a substantially uniform first density. As shown, first and second surfaces of tufted regions **44** coincide with first **41** and second **42** surfaces of pad **40.** As such, the thickness of pad **40** and tufted regions **44** are the same. Interconnecting channels **46** have channel sidewalls **47** extending away from second surface **42** and into core body **45** of absorbent pad **40** and terminating at first surface **48a** of channel base region **48** extending between channel sidewalls **47.** Channel base regions **48** have a thickness (t_{c}) defined by the distance between first surface **48a** and second surface **48b** of base region **48** and a substantially uniform second density greater than the substantially uniform first density of tufted regions **44.** As used herein, the terms "low density, intermediate density, and high density" are relative terms that are used in comparison to each other and unless specifically quantified herein, are not intended to refer to any specific density or any degree of density.

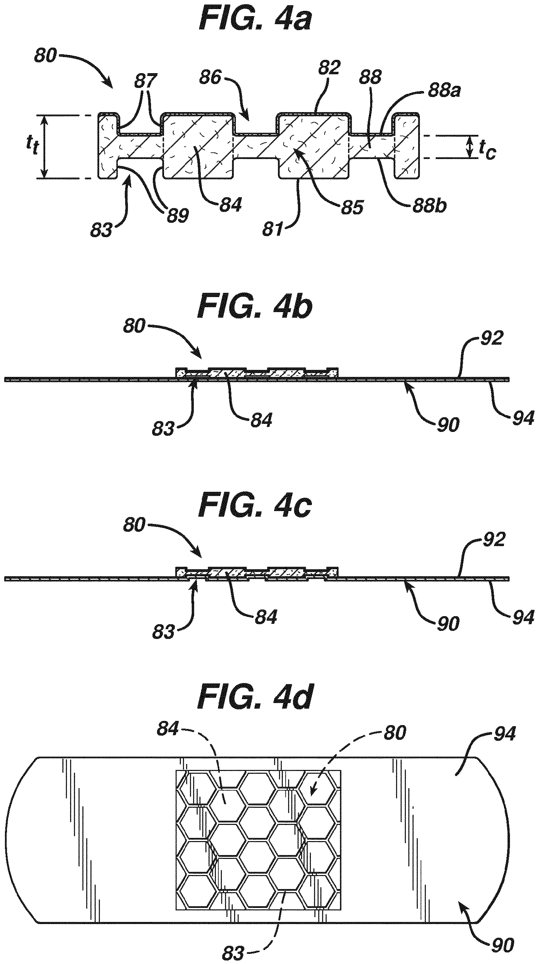

FIG. 4a is a cross-sectional side view of a second embodiment of an absorbent pad used in bandages of the present invention. In this embodiment, absorbent pad **80** has first surface **81,** second surface **82** comprising a covering layer affixed thereto, core body **85,** tufted regions **84,** and interconnecting channels **86** recessed in second surface **82.** The thickness (tₜ) of absorbent pad **80** is defined by the distance between first **81** and second **82** surfaces of absorbent pad **80.** Tufted regions **84** have a thickness of tₜ and a substantially uniform first density. Interconnecting channels **86** recessed in second pad surface **82** have channel sidewalls **87** extending away from second pad surface **82** and into core body **85** of absorbent pad **80** and terminating at first surface **88a** of channel base region **88** extending between channel sidewalls **87.** Interconnecting channels **83** recessed in first pad surface **81** have channel sidewalls **89** extending away from first pad surface **81** and into core body **85** of absorbent pad **80** and terminating at second surface **88b** of channel base region **88** extending between channel sidewalls **89.** Channel base regions **88** have a thickness (t_{c}) defined by the distance between first **88a** and second **88b** surfaces of channel base region **88** and a substantially uniform second density greater than the substantially uniform first density of tufted regions **84.**

FIGS. 4b-4d are embodiments of bandages of the present invention comprising absorbent pad **80** depicted in FIG. 4a. As shown in FIG. 4b, absorbent pad **80** is associated to backing layer **90** via adhesive layer **92.** Interconnecting channels **83** and tufted regions **84** are shown, respectively. In this embodiment, the network of interconnected channels **83** may be visible to the user through second surface **94** of backing layer **90,** for example where backing layer **90** is transparent or translucent, although such visibility is not required. As shown in FIG. 4c, absorbent pad **80** is associated to backing layer **90** via adhesive layer **92.** Channels **83** and tufted regions **84** are shown, respectively. In this embodiment, backing layer **90** conforms to recessed channels **83** such that backing layer **90** includes the network of interconnecting channels **83** recessed in second surface **94** of backing layer **90.** As such, the network of interconnected channels **83** recessed in backing layer **90** is visible to the user. FIG. 4d is a view of the embodiment depicted in FIG. 4c as viewed perpendicular to surface **94** of backing layer **90.** In this embodiment, the densification pattern formed by tufted regions **84** and channels **83** is visible to the user.

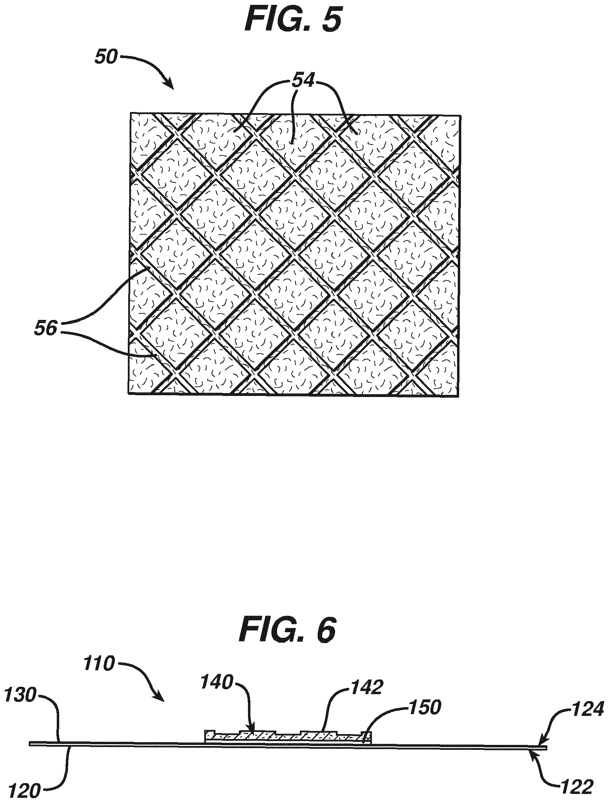

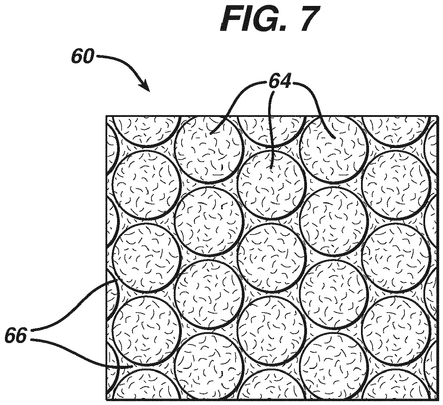

FIGS. 5 and 7 represent additional embodiments of pad densification patterns of absorbent pads used in bandages of the present invention. In FIG. 5, absorbent pad **50** has tufted regions **54** separated by a network of recessed, interconnecting channels **56.** Tufted regions **54** have a diamond shape when viewed perpendicular to the second surface of the absorbent pad. In FIG. 7, absorbent pad **60** has tufted regions **64** separated by a network of recessed, interconnecting channels **66.** Tufted regions **64** have a circular shape when viewed perpendicular to the second surface of the absorbent pad.

Though the tufted regions of absorbent pad embodiments shown have hexagonal, diamond, or circular shapes, it is to be understood that the shape of the tufted regions may be selected from the group consisting of elliptical, ovoid, circular and polygonal. Polygonal includes shapes which have 4 to 8-sides. Where tufted regions are polygonal in shape the channels are linear. When tufted regions are elliptical, ovoid, or circular the channels are non-linear. Though shown as uniform in FIGS. 3 and 5, the length of the sides of the polygonal tufted region may differ.

Yet another embodiment of an adhesive bandage of the present invention is shown in cross-sectional side view in FIG. 6. Adhesive bandage **110** comprises backing layer **120** having a first surface **122** and opposing second surface **124.** Adhesive layer **130** is disposed on second surface **124** of backing layer **120.** Intermediate layer **150** is disposed on adhesive layer **130** so as to achieve bonding between backing layer **120** and intermediate layer **150.** Absorbent pad **140** is associated with intermediate layer **150.**

Though FIG. 6 shows adhesive layer **130** disposed on the entirety of second surface **124** of backing layer **120,** it is to be understood that adhesive layer **130** may be disposed on a portion of second surface **124** of backing layer **120,** as discussed herein above. Absorbent pad **140** is disposed on a portion of intermediate layer **150** and associated with intermediate layer **150** so as to achieve bonding between intermediate layer **150** and absorbent pad **140,** thus also associating pad **140** to backing layer **120** via intermediate layer **150.** The bond may be in the form of an adhesive, or may be any other known means of bonding, such as by ultrasonic welding. Though FIG. 6 shows absorbent pad **140** disposed on the entirety of intermediate layer **150,** it is to be understood that absorbent pad **140** may be disposed on a portion of intermediate layer **150.**

Absorbent pad **140** and intermediate layer **150** may have various shapes, e.g. rectangular, square, oval, circular, ovoid, oblong, etc., and be sized to cover less area than backing layer **120** so that, in use, adhesive layer **130** is in contact with the user's skin, but preferably does not contact the wound surface.

The absorbent pad may be a fibrous matrix comprising an organized network selected from the group consisting of threads, yarns, nets, laces, felts and nonwovens. A preferred method of making the absorbent pad is known to one skilled in the art as the wet lay process of forming nonwovens.

Embossing may be performed ultrasonically through an ultrasonic system which includes a nip roll for providing tension to the web, an engrave/machined roll constructed of hardened steel, and an ultrasonic horn. The ultrasonic horn and engrave/machined roll are set to a positioned so that the roll and horn would have no gap if the material being embossed was not between the rolls. The web would have a consistent and maintained tension, embossing the web with an ultrasonic system.

Alternatively, embossing may be performed using heat and pressure through a heated roll system which includes a nip roll for providing tension to the web, an engrave/machined roll constructed of hardened steel, and an anvil roll. Heat is applied in this area to raise the temperature of the substrate. The anvil roll and engrave/machined roll are set to a position so that the roll and anvil would have no gap if the material being embossed was not between the rolls. The web would have a consistent and maintained tension, embossing the web with an engrave/machined roll and anvil.

The process of manufacturing the adhesive bandage of the present invention may be any of those conventionally known to produce adhesive bandages. The backing layer, absorbent pad, and adhesive layer can be obtained by any methods available at present. For example, an extrusion process may be used for obtaining the backing layer. In the same way, the adhesive layer can be made in any known manner. A backing layer as described herein is obtained and an adhesive layer as described herein is applied to the second surface of the support layer. The absorbent pad is then associated with the adhesive bonding layer, thus bonding the absorbent pad to the backing layer.

The adhesive bandages of the invention are ideally suited to deliver one or more active ingredients such as therapeutics to the surface of the skin. When contained in the adhesive bandages of the invention, one or more active ingredients may be contained primarily or exclusively in the absorbent pad of the adhesive bandage. Illustrative classes of active ingredients that may be delivered to the skin via the adhesive bandages of the invention include, but are not limited to, antibiotics, analgesics, antipyretics, antimicrobials, antiseptics, antiallergics, anti-acne, anesthetics, anti-inflammatories, hemostats, cosmetics, vitamins, vasodilators, emollients, pH regulators, antipruritics, counterirritants, antihistamines and steroids. Specific active ingredients that may be delivered to the skin via the dressings of the invention include chlorhexidine, neomycin sulfate, polymyxin-B sulfate, zinc bacitracin, benzalkonium chloride, cetylpyridinium chloride, bupivacaine, tetracaine, cincaine, lidocaine, benzocaine, silver sulfadiazine, hydrocortisone, metandienone, trypsin, tolazoline, heparin, pramoxine, aloe vera, tretinoin, retinol, retinaldehyde, menthol, capsaicin, alpha hydroxy acids and vitamins such as Vitamin E.

While various embodiments of the invention have been set forth above, it will be apparent to those skilled in the art that various modifications and variations can be made in the present invention without departing from the scope or spirit of the invention. Thus, it is intended that the present invention cover such modifications and variations as come within the scope of the appended claims and their equivalents.

While not intending to limit the scope of the invention or claims appended to the specification, the present invention may be better understood with reference to the following examples.

### EXAMPLE 1: Production of Embossed Pads and Bandages.

Absorbent pads were made from 3.7 oz/yd² polypropylene (PP) and 3.7 oz/yd² polyethylene terephthalate (PET) pad stock, respectively. The pads were embossed with a hexagonal pattern of interconnecting channels. Embossing was performed at slower and faster speeds. Some of the absorbent pads were then adhered to polyethylene backing layers to form adhesive bandages. A total of four different pads and four different bandages were formed.

The thickness of tufted regions (tₜ), the thickness of the channel base regions (t_{c}) and the width of the channel base regions (w_{c}), were measured optically using a Mituoyo PH-A14 Profile Projector with a QM-Data 200. Specimens were razor cut orthogonally to the hexagonal pattern. Five measurements were taken for each of the four different pads and four different bandages formed. The depth of the channel base regions (h_{c}) were calculated based upon the thickness of the tufted and channel base regions.

Table 1 shows the maximum and minimum thickness of tufted regions (tₜ), the maximum and minimum thickness of the channel base regions (t_{c}), the width of the channel base regions (w_{c}), and the depth of the channel base regions (h_{c}).

**Table 1: Dimensions of Embossed Pads and Bandages.**

| Material | Min t_{c} | Max t_{c} | Min tₜ | Max tₜ | w_{c} | h_{c} |
|---|---|---|---|---|---|---|
| | (mm) | (mm) | (mm) | (mm) | (mm) | (mm) |
| PP Pad (slow) | 1.004 | 1.080 | 1.570 | 1.967 | 0.334 | 0.963 |
| PP Pad (fast) | 1.092 | 1.204 | 1.228 | 1.296 | 0.318 | 0.204 |
| PET Pad (slow) | 0.149 | 0.192 | 0.927 | 1.253 | 0.408 | 1.104 |
| PET Pad (fast) | 0.824 | 0.916 | 0.918 | 1.293 | 0.3810 | 0.469 |
| PP Bandage (slow) | 0.268 | 0.306 | 0.804 | 0.993 | 00.469 | 0.725 |
| PP Bandage (fast) | 0.701 | 0.884 | 1.064 | 1.470 | 0.3560 | 0.769 |
| PET Bandage (slow) | 0.142 | 0.152 | 1.119 | 1.309 | 0.302 | 1.167 |
| PET Bandage (fast) | 0.928 | 1.165 | 1.097 | 1.133 | .323 | 0.205 |

### EXAMPLE 2: Fluid Absorption of Embossed and Non-Embossed Pads:

Absorbent 1 inch by 1 inch pads were made from 3.7 oz/yd² polypropylene (PP) non-woven pad stock and 3.7 oz/yd² polyethylene terephthalate (PET) non-woven pad stock, as in Example 1. Some absorbent pads were embossed at a speed intermediate to those of Example 1 with a hexagonal pattern of interconnecting channels for testing. A total of four different pads were tested.

Each pad was placed over a 50μL drop of synthetic blood having viscosity less than 50,000 centipoise. A glass slide and weight (200 grams) were placed on each pad for 10 seconds. After 3 minutes, the blood spread area was then measured using image analysis and recorded as the percentage of the pad area. Three specimens were measured for each sample.

Table 2 shows the spread area for pads having a network of interconnecting channels embossed into the surface versus non-embossed pads.

**Table 2: Fluid Spread Area.**

| Specimen | Spread Area % | Std. Dev. |
|---|---|---|
| PP non-embossed | 21 | 4.1 |
| PP embossed | 48 | 0.2 |
| PET non-embossed | 32 | 2.1 |
| PET embossed | 47 | 7.2 |

The table shows that embossing absorbent pads according to the present invention significantly increases the spread area of the synthetic blood.

### EXAMPLE 3: Ointment Spread of Embossed and Non-Embossed Pads.

Absorbent pads made according to Example 2 were tested for Ointment Spread as described below.

A tinted anti-bacterial ointment (0.1g) was placed on each pad. A glass slide and weight (200 grams) were placed on each pad for 10 seconds. The ointment spread area was then measured using image analysis and recorded as the percentage of the pad area. Three specimens were measured for each sample.

Table 3 shows the spread area for pads having a network of interconnecting channels embossed into the surface versus non-embossed pads.

**Table 3: Ointment Spread Area.**

| Specimen | Spread Area % | Std. Dev. |
|---|---|---|
| PP non-embossed | 70 | 2.2 |
| PP embossed | 57 | 0.6 |
| PET non-embossed | 65 | 0.6 |
| PET embossed | 50 | 7.2 |

The table shows that embossing absorbent pads according to the present invention significantly decreases the spread area of the tinted ointment.

### EXAMPLE 4: Fluid Absorption of Embossed and Non-Embossed Pads on Adhesive Bandages.

Absorbent pads made according to Example 2 were placed on a polyethylene laminate backing layer comprising a pressure sensitive adhesive applied thereto. The bandages were tested for fluid absorption as described below.

Each bandage was placed over a 30μL drop of synthetic blood. A glass slide and weight (200 grams) were placed on each bandage for 10 seconds. After 3 minutes, the blood spread area was then measured using image analysis and recorded as the percentage of the bandage area. Three specimens were measured for each sample and compared to a comparative bandage that did not include a pad having a network of interconnecting channels embossed therein. Table 4 shows the spread area for adhesive bandages according to the present invention versus comparative bandages that do not include a pad having a network of interconnecting channels embossed therein.

**Table 4: Fluid Spread area.**

| Specimen | Spread Area % | Std. Dev. |
|---|---|---|
| Control Bandage with non-embossed Pad | 27 | 1.6 |
| Bandage with PET Embossed Pad | 68 | 3.1 |
| Bandage with PP Embossed Pad | 78 | 5.3 |

The table shows that embossing absorbent pads on adhesive bandages according to the present invention significantly increases the spread area of the synthetic blood compared to the comparative bandage.

### EXAMPLE 5: Ointment Spread of Embossed and Non-Embossed Pads on Adhesive Bandages.

Absorbent pads made according to Example 2 were placed on a polyethylene laminate backing layer comprising a pressure sensitive adhesive applied thereto. The bandages were tested for ointment spread area as described below.

A tinted anti-bacterial ointment (0.05g) was placed on each bandage. A glass slide and weight (200 grams) were placed on each bandage for 10 seconds. The ointment spread area was then measured using image analysis, and recorded as the percentage of the pad area. Three specimens were measured for each sample and compared to a comparative bandage that did not include a pad having a network of interconnecting channels embossed therein. Table 5 shows the spread area for adhesive bandages according to the present invention versus comparative adhesive bandages utilizing pads that do not have a network of interconnecting channels embossed therein.

**Table 5: Ointment Spread Area.**

| Specimen | Spread Area % | Std. Dev. |
|---|---|---|
| Control Bandage with non-embossed Pad | 54 | 0.8 |
| Bandage with PET Embossed Pad | 45 | 1.3 |
| Bandage with PP Embossed Pad | 26 | 1.8 |

The table shows that embossing pads on bandages according to the present invention significantly decreases the spread area of the tinted ointment versus the comparative adhesive bandage that does not include a pad having a network of interconnecting channels embossed therein.

## Claims

1. An adhesive bandage for application to abrasions or cuts in the skin, comprising:
a backing layer comprising a first surface and a second surface opposite said first surface; and
an absorbent pad associated with said backing layer, said absorbent pad comprising,
a first surface facing said second surface of said backing layer and having a first surface area,
a second surface opposite said first surface and having a second surface area; and
a core body,
said absorbent pad comprising a plurality of tufted regions surrounded and
separated by a first network of interconnecting channels recessed in said second surface of said absorbent pad, said channels comprising channel sidewalls extending away from said second surface of said absorbent pad and into said core body of said absorbent pad and terminating in a first surface of a channel
base region extending between said channel sidewalls;
wherein said tufted regions have a substantially uniform first density, said channel base region has a substantially uniform second density greater than said substantially uniform first density of said tufted regions and said network of interconnecting channels is visible.

2. The bandage of claim 1 comprising an adhesive layer applied to at least a portion of said second surface of said backing layer.

3. The bandage of claim 1 or claim 2 wherein said second density of said channel base region is about 12 times or less greater than said first density of said tufted regions.

4. The bandage of any of claims 1 to 3 wherein said substantially uniform second density of said channel base region is from about 0.11 g/cc to about 0.96 g/cc.

5. The bandage of any of claims 1 to 4 wherein said substantially uniform first density of said tufted regions is from about 0.082 g/cc to about 0.17 g/cc.

6. The bandage of any of claims 1 to 5 wherein said channels have a width of from about 0.2 to about 0.6 millimeters and a depth from about 0.02 to about 2 millimeters.

7. The bandage of any of claims 1 to 6 wherein said absorbable pad is not degradable in water.

8. The bandage of any of claims 1 to 7 wherein said first network of interconnecting channels comprises from about 5 to about 20 percent of said second surface area of said second surface of said absorbent pad.

9. The bandage of any of claims 1 to 8 wherein said absorbent pad comprises fibers selected from the group consisting of natural and synthetic materials.

10. The bandage of any of claims 1 to 9 further comprising an intermediate layer disposed between said backing layer and said absorbent pad.

11. The bandage of any of claims 1 to 10 wherein the shape of said tufted regions is selected from the group consisting of polygonal, elliptical, ovoid and circular.

12. The bandage of any of claims 1 to 11 wherein said channels are non-linear and the shape of said tufted regions is selected from the group consisting of elliptical, ovoid and circular.

13. The bandage of claim 11 wherein said channels are linear and the shape of said tufted regions is polygonal.

14. The bandage of any of claims 1 to 13 further comprising a second network of interconnecting channels recessed in said first surface of said absorbent pad, said tufted regions being surrounded and separated by said second network of interconnecting channels recessed in said first surface of said absorbent pad, said channels in said second network of interconnecting channels comprising channel sidewalls extending away from said first surface and into said core body of said absorbent pad and terminating in a second surface of said channel base region opposite said first surface of said channel base region and extending between said channel sidewalls of said second network.

15. The bandage of any of claims 1 to 14 wherein said thickness of said absorbent pad is from about 0.3 millimeters to about 2.5 millimeters.

16. The bandage of any of claims 1 to 15 wherein the thickness of said base channel region is from about 0.1 millimeters to about 1.5 millimeters.

17. The bandage of claim 14 wherein said second network of interconnecting channels is visible.

18. The bandage of any of claims 1 to 17 wherein said second surface of said absorbent pad comprises a covering layer.
